# EUROPEAN PATENT APPLICATION

(11) **EP 4 834 872 A1**
(43) Date of publication of application: **04.11.2026**
(21) Application number: 24912629.3
(22) Date of filing: 19.12.2024
(51) Int. Cl.: C07C 29/152, C02F 3/12, C02F 3/28, C07B 61/00, C07C 31/04, C25B 1/04, C25B 9/00

(54) **METHANOL PRODUCTION METHOD AND METHANOL PRODUCTION DEVICE**

(30) Priority: 25.12.2023 JP 2023218416
(71) Applicant: MITSUBISHI GAS CHEMICAL COMPANY, INC., Chiyoda-ku Tokyo 100-8324 (JP)
(72) Inventor: SHIBATA, Akihiro, Tokyo 100-8324 (JP); ABE, Takanori, Tokyo 100-8324 (JP); KAKIMI, Atsushi, Tokyo 100-8324 (JP)
(74) Representative: Vossius & Partner Patentanwälte Rechtsanwälte mbB
(86) International application number: PCT/JP2024/045017
(87) International publication number: WO 2025/142740

(57) **Abstract**

A method for producing methanol, including a step (A) of obtaining a synthesis gas containing at least carbon dioxide and hydrogen, a step (B) of reacting the synthesis gas in the presence of a catalyst to obtain a methanol mixture, a step (C) of distilling the methanol mixture to separate methanol, distillation waste liquid, and distillation wastewater, respectively and a step (D) of performing an organic matter decomposition treatment on the distillation waste liquid and/or the distillation wastewater to obtain decomposed gas and treatment water.

## Description

### Technical Field

The present invention relates to a method for producing methanol and an apparatus for producing methanol.

### Background Art

In conventional methods for industrially producing methanol using natural gas as a raw material, methanol is synthesized from a synthetic gas that is obtained by adding steam to the natural gas (main component: methane), which is a raw material, to cause steam reforming. Since a reaction product obtained by the synthesis is a methanol mixture containing a component other than methanol (hereinafter, also referred to as crude methanol), the crude methanol is purified by a distillation step, and purified methanol is obtained. In the distillation step, since a certain amount of distillation waste liquid and distillation wastewater are discharged to maintain the methanol purity, the reuse thereof has been proposed in view of reducing environmental load. However, in particular, in the production of methanol containing carbon dioxide as a main raw material, it is difficult to recycle an organic matter that is contained in distillation waste liquid as it is in the raw material system, and there is a need to decompose the organic matter into hydrogen, carbon monoxide, or carbon dioxide and reuse those.

For example, in Patent Literature 1, for the purpose of optimizing a method for producing methanol by a reaction between carbon dioxide and hydrogen regarding the efficiency, energy consumption, and the purities of an off-gas stream, a wastewater stream, and a product to be obtained, disclosed is a method for producing methanol in which a predetermined methanol-containing product stream is subsequently supplied to at least one distillation step, at least one component, particularly, water, is separated and removed from the methanol-containing product stream in the at least one distillation step, a gas stream containing at least one separated and removed volatile component is wholly or only partially discharged from a system as an off-gas, and/or this separated and removed gas stream or a part of this gas stream is recycled in the methanol synthesis reaction.

### Citation List

### Patent Literature

Patent Literature 1: Japanese Translation of PCT International Application Publication No. 2019-527691

### Summary of Invention

### Technical Problem

However, in Patent Literature 1, while a part of a fraction separated from methanol or the like is recycled and used in the methanol synthesis reaction, the recycling target is only the volatile component, and it is difficult to say that distillation waste liquid or the like can be sufficiently recycled.

The present invention has been made in consideration of the above-described circumstance, and an object of the present invention is to provide a method for producing methanol, in which the carbon collection rate from distillation waste liquid is excellent and the environmental load is low.

### Solution to Problem

As a result of intensive studies for achieving the above-described object, the present inventors have found that the present invention having the following configuration makes it possible to provide a method for producing methanol, in which the carbon collection rate from distillation waste liquid is excellent and the environmental load is low and completed the present invention. That is, the present invention is as described below.

[1] A method for producing methanol, comprising:
   a step (A) of obtaining a synthesis gas containing at least carbon dioxide and hydrogen;
   a step (B) of reacting the synthesis gas in the presence of a catalyst to obtain a methanol mixture;
   a step (C) of distilling the methanol mixture to separate methanol, distillation waste liquid, and distillation wastewater, respectively; and
   a step (D) of performing an organic matter decomposition treatment on the distillation waste liquid and/or the distillation wastewater to obtain decomposed gas and treatment water.
[2] The method for producing methanol according to [1], further comprising:
   a step (E) of electrolyzing at least one of the distillation wastewater and the treatment water to obtain electrolyzed hydrogen and electrolyzed oxygen.
[3] The method for producing methanol according to [2], wherein the electrolyzed hydrogen is used in the step (A).
[4] The method for producing methanol according to any one of [1] to [3], further comprising:
   a step (F) of using the decomposed gas in the step (A).
[5] The method for producing methanol according to any one of [1] to [4], wherein the organic matter decomposition treatment is an anaerobic treatment.
[6] The method for producing methanol according to any one of [1] to [5], further comprising:
   a step (G) of combusting the decomposed gas to recover heat.
[7] The method for producing methanol according to any one of [2] to [6], wherein, in the step (G), the oxygen obtained in the step (E) is used for combustion.
[8] The method for producing methanol according to any one of [1] to [7], wherein the organic matter decomposition treatment is an aerobic treatment.
[9] The method for producing methanol according to any one of [1] to [8], further comprising:
   a step (H) of collecting carbon dioxide from the decomposed gas.
[10] An apparatus for producing methanol, comprising:
   a synthesis gas preparation unit;
   a methanol synthesis unit;
   a distillation unit; and
   an organic matter decomposition treatment unit.
[11] The apparatus for producing methanol according to [10], further comprising:
   an electrolysis unit,
   wherein electrolyzed hydrogen that is obtained in the electrolysis unit is used in the synthesis gas preparation unit.

### Advantageous Effect of Invention

The present invention makes it possible to provide a method for producing methanol in which the carbon collection rate from distillation waste liquid is excellent and the environmental load is low.

### Brief Description of Drawings

[FIG.1] FIG. 1 is a schematic view showing an example of a production apparatus that is used in a method for producing methanol of the present embodiment.
[FIG. 2] FIG. 2 is a schematic view showing another example of the production apparatus that is used in the method for producing methanol of the present embodiment.
[FIG. 3] FIG. 3 is a schematic view showing one example of a production apparatus that is used in a method for producing methanol corresponding to a comparative example.
[FIG. 4] FIG. 4 is a schematic view showing an example of an anaerobic treatment unit of the present embodiment.
[FIG. 5] FIG. 5 is a schematic view showing examples of a gasification preparation step of the present embodiment.
[FIG.6] FIG. 6 is a schematic view showing more examples of the gasification preparation step of the present embodiment.

### Description of Embodiments

Hereinafter, an embodiment for carrying out the present invention (hereinafter, simply referred to as "the present embodiment") will be described in detail with reference to drawings as necessary, but the present invention is not limited to the following embodiment. The present invention can be modified in a variety of manners within the scope of the gist thereof. In the drawings, the same elements will be given the same reference sign, and duplicate description will be omitted. In addition, positional relationships, such as up, down, right, and left, will be based on the positional relationship shown in the drawings unless particularly otherwise described. Furthermore, dimensional ratios of the drawings are not limited to ratios shown in the drawings.

### [Method for producing methanol]

A method for producing methanol, including
a step (A) of obtaining a synthesis gas containing at least carbon dioxide and hydrogen,
a step (B) of reacting the synthesis gas in the presence of a catalyst to obtain a methanol mixture,
a step (C) of distilling the methanol mixture to separate methanol, distillation waste liquid, and distillation wastewater, respectively and
a step (D) of performing an organic matter decomposition treatment on the distillation waste liquid and/or the distillation wastewater to obtain decomposed gas and treatment water.

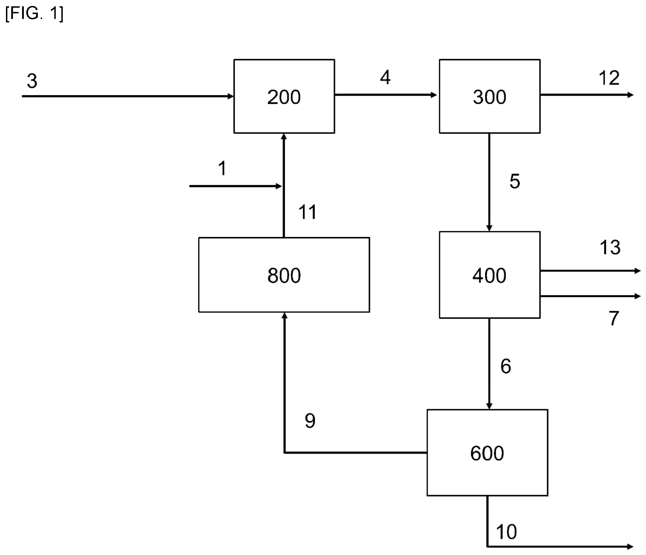

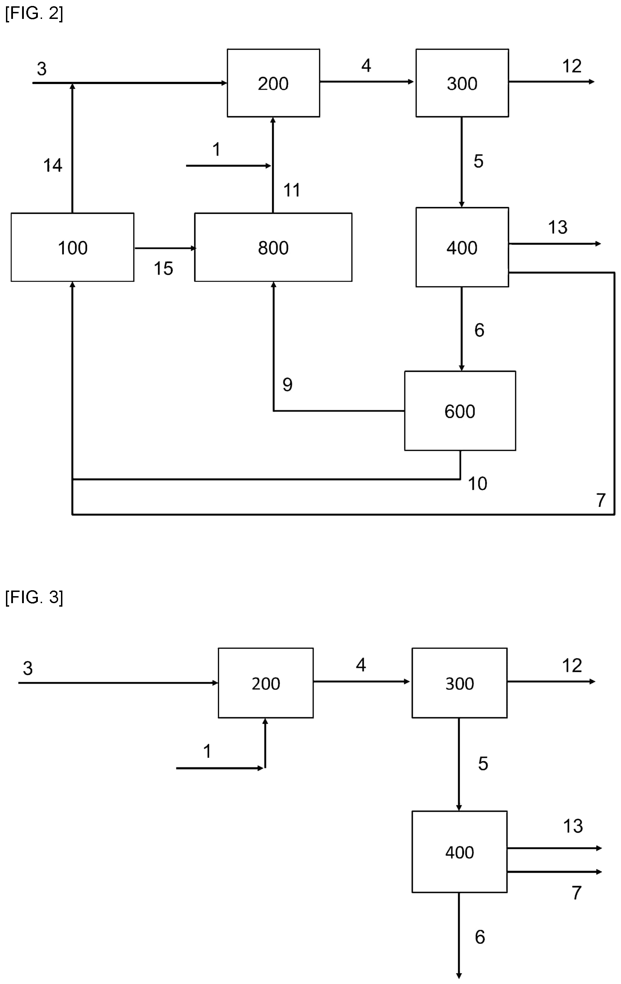

Hereinafter, the method for producing methanol of the present embodiment will be described using apparatuses for producing methanol of FIGS. 1 and 2. However, the method for producing methanol of the present embodiment is not limited to embodiments in which the apparatuses for producing methanol of FIGS. 1 and 2 are used.

### [Step (A)]

The step (A) is a step of obtaining a synthesis gas containing at least carbon dioxide and hydrogen with a synthesis gas preparation unit 200 as shown in FIGS. 1 and 2. In the step (A), a carbon dioxide-containing gas 1 is supplied to a synthesis gas preparation unit 200 together with hydrogen 3 and turned into a synthesis gas 4. The synthesis gas 4 is a gas that is used for the synthesis of methanol and is a gas containing hydrogen, carbon monoxide, and carbon dioxide as main components.

The hydrogen is not particularly limited; however, for example, hydrogen obtained by using renewable energy is preferably used from the viewpoint of decreasing the amount of carbon dioxide discharged. More specific examples thereof include electrolyzed hydrogen, which will be described below, blue hydrogen, such as by-product hydrogen from an oil refinery facility, by-product hydrogen derived from a chemical process, and by-product hydrogen for which CCS is jointly used, hydrogen from which a gas composition has been adjusted with PSA or the like, and hydrogen that is obtained by steam reforming.

The step (A) may include a gasification preparation step of gasifying and/or combusting an organic matter and/or a hydrocarbon-containing gas to obtain the synthesis gas 4.

The organic matter is not particularly limited, and examples thereof include waste plastics, biomass, and organic waste.

The above-described hydrocarbon-containing gas is not particularly limited, and examples thereof include purified gases and fossil fuel gases. The purified gases are not particularly limited, and examples thereof include methane, ethane, propane, butane, and gas mixtures thereof. Examples of the fossil fuel gases include natural gas (NG) containing methane as a main component, liquefied petroleum gas (LPG), and naphtha.

The gasification preparation step includes a gasification step and may further include one or more steps of a reforming step, a gas cleaning step, a carbon dioxide separation step, a hydrogen separation step, a shift reaction step, and a reverse shift reaction step.

Here, the gasification step is not particularly limited, a conventionally well-known method can be used, and examples thereof include steps in which a pneumatic type gasifier is used and steps in which an oxygen-steam type gasifier is used. These can be further classified into a fixed bed type and a fluidized bed type, but both types can be used for those steps.

The reforming step is not particularly limited, and examples thereof include steam methane reforming (SMR), autothermal reforming (ATR), two-stage reforming (SMR + ATR), and a partial oxidation method. In addition, in a case where the hydrocarbon-containing gas is natural gas or naphtha, the step (A) may include a pre-reforming step of providing a pre-reformer upstream of a reforming unit and reforming the natural gas or the naphtha at approximately 500°C to produce a methane-rich gas (not particularly limited; however, for example, the CH₄ content ratio is approximately 30 to 50 mol%). As the reforming temperature, a conventionally well-known temperature can be used, there is no particular limitation, and the reforming temperature can be set to, for example, 750°C to 1000°C. In addition, a catalyst may be used for the reforming, a conventionally well-known catalyst may be used as such a catalyst, there is no particular limitation, and examples thereof include nickel-based catalysts.

The gas cleaning step is not particularly limited, a conventionally well-known method can be used, and examples thereof include methods in which a bubble stirring tank, a spray tower, a wetted wall tower, or a packed tower is used.

The carbon dioxide separation step is not particularly limited, a conventionally well-known method can be used, and examples thereof include methods in which a carbon dioxide separation apparatus using a pressure swing adsorption method, a temperature swing adsorption method, or a membrane separation method is used.

The hydrogen separation step is not particularly limited, a conventionally well-known method can be used, and examples thereof include methods in which a hydrogen separation apparatus using a pressure swing adsorption method, a temperature swing adsorption method, or a membrane separation method is used.

The shift reaction step and the reverse shift reaction step are not particularly limited, conventionally well-known methods can be used, and the shift reaction step and the reverse shift reaction step can be performed, for example, in the presence of a catalyst. As the catalyst, a conventionally well-known catalyst can be used, there is no particular limitation, and examples thereof include transition metal oxides, such as iron oxide (Fe₃O₄), or platinum. Here, the shift reaction step refers to a step of generating carbon dioxide and hydrogen from carbon monoxide and steam, and the reverse shift reaction step refers to a step of performing a reverse reaction of the shift reaction (generating carbon monoxide and steam from carbon dioxide and hydrogen).

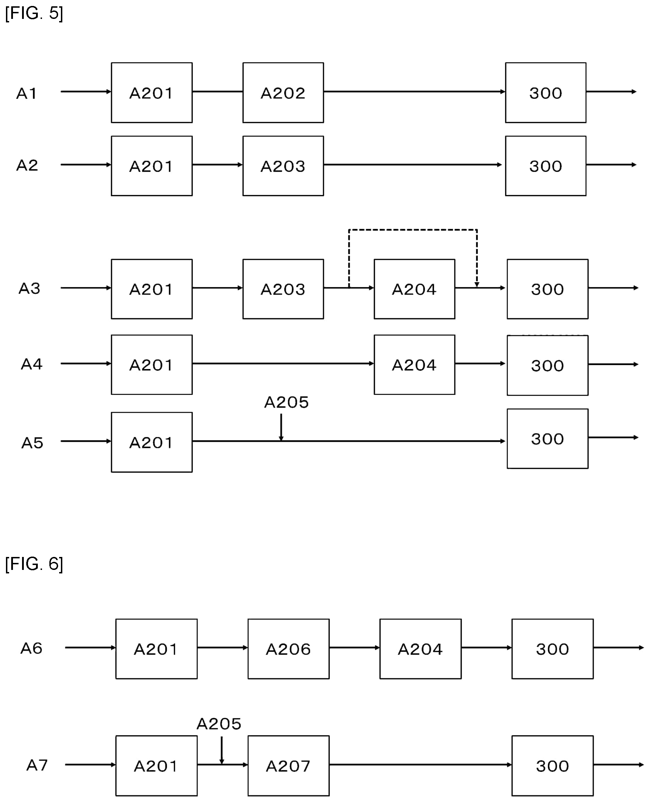

Specific examples of the gasification preparation step are not particularly limited, and, for example, configurations in FIGS. 5 and 6 can be used.

A gasification preparation step A1 in FIG. 5 is a step of obtaining a synthesis gas by undergoing a gasification step A201 and a gas cleaning step A202 in order.

A gasification preparation step A2 in FIG. 5 is a step of obtaining a synthesis gas by undergoing the gasification step A201 and a reforming step A203 in order.

A gasification preparation step A3 in FIG. 5 is a step of obtaining a synthesis gas by undergoing the gasification step A201, the reforming step A203, and a carbon dioxide separation step A204 as necessary in order.

A gasification preparation step A4 in FIG. 5 is a step of obtaining a synthesis gas by undergoing the gasification step A201 and the carbon dioxide separation step A204 in order.

A gasification preparation step A5 in FIG. 5 is a step of obtaining a synthesis gas by undergoing the gasification step A201 and a hydrogenation step A205 in order.

A gasification preparation step A6 in FIG. 6 is a step of obtaining a synthesis gas by undergoing the gasification step A201, a shift reaction step A206, and the carbon dioxide separation step A204 in order.

A gasification preparation step A7 in FIG. 6 is a step of obtaining a synthesis gas by undergoing the gasification step A201, the hydrogenation step A205, and a reverse shift reaction step A207 in order.

### [Step (B)]

The step (B) in the method for producing methanol of the present embodiment is a step of supplying the synthesis gas 4 to a methanol synthesis unit 300 and reacting the synthesis gas in the presence of a catalyst to obtain a crude methanol 5 as shown in FIGS. 1 and 2. A part of a purged gas 12 that is generated as a by-product in association with the generation of the crude methanol may be supplied to the synthesis gas preparation unit 200 or a desulfurization unit that can be disposed upstream thereof.

In the step (B), a reaction mixture that is obtained by the reaction is cooled and then separated into gas and liquid, whereby the crude methanol 5 can be obtained as a liquid phase, and the purged gas 12 containing an unreacted gas or the like can be obtained as a gas phase. As a method for the gas-liquid separation, a conventionally well-known method can be used, there is no particular limitation, and for example, a high-pressure separator can be used.

While depending on the conditions of the methanol synthesis reaction, the purged gas is a gas mixture that may contain hydrogen, carbon monoxide, carbon dioxide, methane, nitrogen, and the like as a composition thereof. At least a part of the purged gas is preferably supplied to a shift reaction unit and/or a boiler. This makes it possible to further reduce the amount of carbon dioxide discharged.

The gas temperature at the entry of the methanol synthesis unit 300 is set as appropriate depending on the kind or amount of the catalyst, the shape and reaction pressure of a reactor, and the like, and is preferably 170°C to 260°C, more preferably 170°C to 220°C, and still more preferably 170°C to 200°C. When the entry gas temperature is 170°C or higher, there is a tendency that the reactivity improves, and when the entry gas temperature is 260°C or lower, there is a tendency that the equipment cost can be reduced.

The gas pressure at the entry of the methanol synthesis unit 300 is preferably 4.9 to 14.7 MPaG, more preferably 5.0 to 11.0 MPaG, and still more preferably 5.0 to 10.0 MPaG. When the entry gas pressure is 4.9 MPaG or higher, there is a tendency that the reactivity improves, and when the entry gas pressure is 14.7 MPaG or lower, there is a tendency that the production efficiency increases.

For the synthesis gas 4 that is supplied to the methanol synthesis unit 300, the mol% relationship (M value) among CO, carbon dioxide, and hydrogen that is calculated from the following equation:$M value = \left(hydrogen mol%\right) / \left(2\times CO mol%+3\times carbon dioxide mol%\right)$ is preferably 0.9 to 5.0, more preferably 0.9 to 3.0, still more preferably 0.9 to 2.0, and particularly preferably 1.0 to 1.5. When the M value is 1.3 or more, there is a tendency that the amount of a by-product decreases, and when the M value is 5.0 or less, there is a tendency that the carbon yield of methanol synthesis is excellent.

Here, the carbon yield of methanol synthesis means the ratio of the molar flow rate of methanol that is generated in the methanol synthesis unit 300 to the total amount of the molar flow rate of carbon monoxide and the molar flow rate of carbon dioxide that are contained in the synthesis gas 4 that is supplied to the methanol synthesis unit 300.

The reaction temperature in the methanol synthesis unit 300 is preferably 200°C to 300°C, more preferably 200°C to 280°C, and still more preferably 200°C to 270°C from the viewpoint of maintaining the reactivity, suppressing a by-product, and protecting the catalyst.

The kind of the methanol synthesis unit 300 is not particularly limited, but is preferably, for example, a unit having a mechanism capable of controlling the reaction temperature. Specific examples thereof include a heat exchange-type reactor and a quench-type adiabatic reactor. The heat exchange-type reactor is not particularly limited, and examples thereof include a multi-tube heat exchange-type reactor and a radial flow-type reactor.

In the case of using the multi-tube heat exchange-type reactor, the reaction temperature is controlled by indirect heat exchange with pressurized boiling water, and saturated vapor (steam) is obtained. The boiling water is circulated in a steam drum and the shell side of the reactor, and the steam is collected from a steam drum. Steam that is obtained with this synthesis system is preferably used as a heat source for the purification process of a methanol solution that is present downstream of a synthesis process. The pressurized boiling water is preferably 220°C to 260°C.

In the case of employing the adiabatic reactor, the reactor has one or more catalyst layers therein, and in a case where the reactor has two or more layers, a part of a synthetic reactor supply gas is branched as a cooling gas of an interlayer and supplied as a quench gas, whereby the reaction temperature is controlled, an evaporator is installed in a reactor exit gas as a heat collector to collect steam, and the steam may be, similarly, used as the heat source for the purification process of the methanol solution downstream.

The catalyst that is used for the synthesis is preferably a methanol synthesis catalyst containing copper atoms and zinc atoms as essential components. Such a catalyst is reduced from an oxide state by a reducing gas, for example, hydrogen, carbon monoxide, or a gas mixture thereof, whereby copper is activated, and the catalyst has a catalyst activity. The catalyst may contain, in addition to the copper atoms and the zinc atoms, aluminum atoms and/or chromium atoms as a third main component. The catalyst containing copper and zinc as essential components can be prepared by a well-known method. Such a catalyst can be prepared by, for example, methods described in Japanese Patent Publication No. 51-44715, Japanese Patent No. 2695663, Japanese Patent Publication No. 6-35401, Japanese Patent Laid-Open No. 10-272361, and Japanese Patent Laid-Open No. 2001-205089.

A preferable catalyst is a methanol synthesis catalyst containing copper atoms and zinc atoms in an atomic ratio (copper/zinc) of 2.0 to 3.0 and containing aluminum atoms. Such a catalyst is not particularly limited, and examples thereof include catalysts prepared by a method described in Japanese Patent Laid-Open No. 8-299796 and a catalyst described in International Publication No. WO 2011/048976.

Specific examples of the preferable catalyst include catalysts used in examples and comparative examples, for example, Example 2 and Example 3, of International Publication No. WO 2011/048976. In addition, a more preferable atomic ratio (copper/zinc) of the copper atoms and the zinc atoms in the catalyst is within a range of 2.1 to 3.0. A methanol synthesis catalyst containing, in addition to those, 3 to 20 mass% of alumina is still more preferable. As described above, such a catalyst is not particularly limited and can be prepared by a method described in International Publication No. WO 2011/048976. More specifically, the catalyst is prepared by, for example, a production method having a step of mixing an aqueous solution containing copper, an aqueous solution containing zinc, and an alkali aqueous solution to generate a precipitate containing copper and zinc, a step of mixing the obtained precipitate and an alumina hydrate having a pseudo-boehmite structure to obtain a mixture, and a step of molding the obtained mixture so that the density reaches 2.0 to 3.0 g/mL. Here, examples of a molding method include tableting, extrusion, and rolling granulation. The catalyst that is used in the present embodiment is not limited to the above-described catalysts and catalysts prepared by the above-described preparation methods and may be a different catalyst having an equivalent methanol synthesis activity.

### [Step (C)]

The method for producing methanol of the present embodiment includes the step (C) of distilling the crude methanol 5 obtained in the step (B) with a distillation unit 400 to separate methanol 13, distillation waste liquid (side cut liquid) 6, and distillation wastewater (bottoms) 7, respectively as shown in FIGS. 1 and 2. The methanol in the present embodiment means purified methanol. Here, the distillation wastewater means a liquid component containing distillation waste liquid (side cut liquid) and may further include distillation wastewater (bottoms). The distillation waste liquid means a liquid component containing concentrated methanol that is drawn out to the outside of a distillation tower in each stage of the tower, concentrated alcohols other than methanol, and water. The distillation wastewater means a liquid component that is drawn out from the tower bottom of the distillation tower to the outside of the tower and is mainly composed of water.

As the step (C), a conventionally well-known method can be used, there is no particular limitation, and examples thereof include distillation for which a distillation tower including a reboiler and a condenser is used. In that case, highly pure methanol is obtained from the tower top by distilling a methanol mixture.

In the step (C), steam collected in the step (A) or the like may be used. The use of the steam makes it possible to absorb or adsorb a fluid that is discharged from the tower top in the case of, for example, distilling the methanol mixture using a distillation tower. This makes it possible to further reduce the amount of carbon dioxide discharged.

In the step (C), it is also possible to use heat collected in the step (A). Such heat is not particularly limited and can be used as heat necessary in the reboiler in the case of, for example, distilling the methanol mixture using a distillation tower. This makes it possible to further reduce the amount of carbon dioxide discharged. Renewable energy may also be supplied from the outside and used as heat.

### [Step (D)]

The method for producing methanol of the present embodiment includes the step (D) of performing an organic matter decomposition treatment on the distillation waste liquid 6 and/or the distillation wastewater 7 obtained by the step (C) with an organic matter decomposition treatment unit 600 to obtain decomposed gas 9 and treatment water 10 as shown in FIGS. 1 and 2.

The organic matter decomposition treatment in the step (D) is not particularly limited, and examples thereof include an aerobic treatment (active sludge method), an anaerobic treatment, a hydrothermal gasification treatment, and a combustion treatment. While varying with the kind of organic matter decomposition, the step (D) may be provided with a pH adjustment step before the supply of the distillation waste liquid 6 to the organic matter decomposition treatment unit 600.

The aerobic treatment refers to a microbial treatment that is performed under a condition where oxygen is sufficiently present and is widely performed in a water treatment plant and the like. Examples of the aerobic treatment are not particularly limited and include an active sludge method. The aerobic treatment can be relatively easily performed in an open system and is thus preferably used.

The anaerobic treatment refers to a treatment for decomposing an organic matter using a microbe under an atmosphere where oxygen is absent or deficient. The anaerobic treatment is preferably used since the amount of sludge generated can be reduced, and most of the purified gas is hydrocarbon and carbon dioxide.

The hydrothermal gasification treatment refers to a treatment for decreasing the molecular weight of an organic matter in an aqueous solution using a catalyst in a high-temperature and high-pressure liquid phase. The catalyst is not particularly limited, a conventionally well-known catalyst can be used, and, for example, a catalyst carrying a transition metal, such as nickel, can be used. The temperature is not particularly limited and may be set to, for example, 250°C to 300°C, and the pressure is not particularly limited and may be set to, for example, 10 to 20 MPa.

The obtained decomposed gas 9 is not particularly limited, but is preferably used in the step (A), and may be supplied to the synthesis gas preparation unit 200 through a combustion/heat recovery unit 800 as shown in FIGS. 1 and 2.

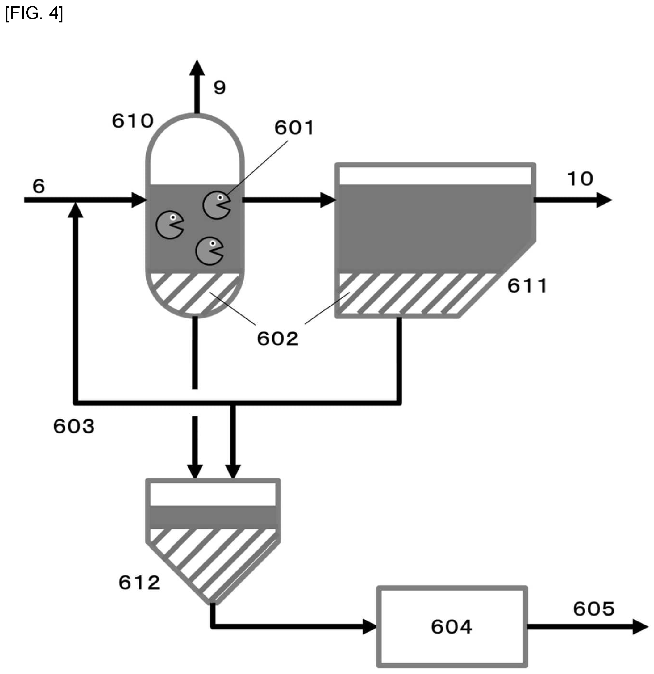

The organic matter decomposition treatment is not particularly limited, a conventionally well-known method can be used, and examples thereof include, in a case where the organic matter decomposition treatment is an anaerobic treatment, a method in which an acid formation reaction tank by an acid former is used and a method in which a methane generation reaction tank by a methanogen is used. FIG. 4 shows a method in which a methane generation reaction tank by a methanogen is used, and the distillation waste liquid 6 that is injected into the system is first injected into a methane generation reaction tank 610 where a microbe is present. There, the organic matter is decomposed by microbes 601, and a digestion gas is generated as the decomposed gas 9. The digestion gas contains methane, carbon dioxide, or the like and can be thus supplied to and used in the combustion/heat recovery unit or the synthesis gas preparation unit 200. A part of a solvent in the methane generation reaction tank is sent to a precipitation tank 611 and taken out as the treatment water 10. Returned sludge 603 that is a part of sludge 602 accumulated in the methane generation reaction tank 610 and the precipitation tank 611 is supplied to the methane generation reaction tank 610 again as an organic matter, and the remainder is sent to a sludge concentration tank 612, subjected to a concentration treatment, then, subjected to a dehydration treatment with a sludge dehydration unit 604, and collected as dehydrated sludge 605.

### [Step (G)]

The method for producing methanol of the present embodiment preferably further includes the step (G) of combusting the decomposed gas 9 that is obtained by the organic matter decomposition treatment to recover heat. Heat recovered in the combustion/heat recovery unit 800 can be recycled as a heat source in the synthesis gas preparation unit 200 or the like, and carbon dioxide generated by the combustion can be supplied to the synthesis gas preparation unit 200 as a recycling gas 11. As oxygen that is used for combustion, the electrolyzed oxygen that is obtained in the step (E), which will be described below, can be used for the combustion.

In the method for producing methanol of the present embodiment, the molar flow rate of a carbon atom that is contained in the decomposed gas 9 is preferably 70 mol% or more, more preferably 80 mol% or more, still more preferably 90 mol% or more, and particularly preferably 95 mol% or more based on the molar flow rate of a carbon atom that is contained in the distillation waste liquid 6 and the distillation wastewater 7 from the viewpoint of the carbon collection rate from the distillation waste liquid and/or the distillation wastewater.

### [Step (H)]

The method for producing methanol of the present embodiment preferably further includes the step (H) of collecting carbon dioxide from the decomposed gas. As a method for separating carbon dioxide, a conventionally well-known method can be used, there is no particular limitation, and examples thereof include a physical absorption method, a physical adsorption method, a chemical absorption method, a chemical adsorption method, a membrane separation method, a cryogenic separation method, and an electric adsorption method.

### [Step (E)]

The method for producing methanol of the present embodiment preferably further includes the step (E) of electrolyzing at least one of the distillation wastewater 7 and the treatment water 10 with an electrolysis unit 100 to obtain electrolyzed hydrogen 14 and electrolyzed oxygen 15 as shown in FIG. 2.

The electrolyzed hydrogen 14 preferably joins the hydrogen 3 and is supplied to the synthesis gas preparation unit and used in the step (A), and the electrolyzed oxygen 15 is preferably supplied to the combustion/heat recovery unit 800 and used for heat recovery.

As the electrolysis, a conventionally well-known method can be used, there is no particular limitation, and, for example, the electrolysis can be performed by supplying power that is supplied from a power supply to an electrolysis tank composed of two electrodes (an anode and a cathode) that are separated with an ion exchange membrane.

### [Desulfurization step]

The method for producing methanol of the present embodiment may include a desulfurization step with a desulfurization unit. The desulfurization step is a step of supplying the carbon dioxide-containing gas 1 and the recycling gas 11 to the desulfurization unit to obtain a desulfurized gas from which a sulfur content has been removed. The desulfurized gas is supplied to the synthesis gas preparation unit 200. Since a sulfur compound becomes a catalyst poison of the catalyst that is used in the step (A) or the step (B), in a case where the carbon dioxide-containing gas 1 contains a sulfur content, it is preferable to remove the sulfur content in advance in the desulfurization step. As a desulfurization method, a conventionally well-known method can be used, there is no particular limitation, and examples thereof include a dry method in which an adsorption agent or a catalyst is used and a wet method in which an amine-based or different absorption liquid is used. In the case of using the dry method, the operation temperature can be set to 0°C to 400°C while varying with the kind of the sulfur compound that is a removal target or the kind of the catalyst that is employed.

### [Other steps]

The method for producing methanol of the present embodiment may include, aside from the above-described steps, other steps as necessary.

### [Apparatus for producing methanol]

An apparatus for producing methanol of the present embodiment is an apparatus for performing the above-described method for producing methanol, and examples thereof include apparatus shown in FIGS. 1 and 2.

The apparatus for producing methanol of the present embodiment includes the synthesis gas preparation unit 200, the methanol synthesis unit 300, the distillation unit 400, and the organic matter decomposition treatment unit 600. In addition, the apparatus for producing methanol of the present embodiment preferably further includes the electrolysis unit 100, and the electrolyzed hydrogen 14 that is obtained in the electrolysis unit 100 is preferably used in the synthesis gas preparation unit 200. The apparatus for producing methanol of the present embodiment may include other units as necessary.

The synthesis gas preparation unit 200 may include one or more apparatuses or reactors out of a reformer, a gasifier, a gas cleaning apparatus, a carbon dioxide separator, a hydrogen separator, a shift reactor, a reverse shift reactor, and a reforming reactor. Among them, the synthesis gas preparation unit 200 preferably includes a reformer from the viewpoint of improving the carbon yield of methanol synthesis or the like.

The methanol synthesis unit 300 includes a methanol synthesis reactor where the synthesis gas 4 obtained in the synthesis gas preparation unit 200 is reacted in the presence of a catalyst to generate the crude methanol 5 and the purged gas 12 and may include other equipment as necessary.

As the distillation unit 400, a conventionally well-known unit can be used, there is no particular limitation, and examples thereof include distillation towers including a reboiler and a condenser.

The organic matter decomposition treatment unit 600 is not particularly limited, and examples thereof include an anaerobic treatment unit, an aerobic treatment unit, a hydrothermal gasification treatment unit, and a combustion treatment unit. The aerobic treatment unit is not particularly limited as long as the unit is an apparatus having a decomposition tank with an aerobic microbe. The anaerobic treatment unit is not particularly limited as long as the unit is an apparatus having a decomposition tank with an anaerobic microbe. The hydrothermal gasification treatment unit is not particularly limited as long as the unit is an apparatus having a reactor that decreases the molecular weight of an organic matter in an aqueous solution using a catalyst in a high-temperature and high-pressure liquid phase. The combustion treatment unit is not particularly limited as long as the unit is an apparatus having a combustion apparatus.

As the electrolysis unit 100, a conventionally well-known unit can be used, there is no particular limitation, and, for example, an electrolysis tank that is connected to a power supply and composed of two electrodes (an anode and a cathode) that are separated with an ion exchange membrane can be used.

In addition, the apparatus for producing methanol of the present embodiment preferably further includes the desulfurization unit as necessary while not shown in the drawings. The desulfurization unit includes a desulfurizer where a sulfur content is removed from the carbon dioxide gas and may include different equipment as necessary.

### Examples

Hereinafter, the method for producing methanol and apparatus for producing methanol of the present invention will be described in detail with examples and comparative examples, but the present invention is not limited thereto.

As a catalyst that was used for methanol synthesis, any of a catalyst prepared by a method described in Example 1 of Japanese Patent Publication No. 51-44715 (methanol synthesis catalyst A), a catalyst prepared by a method described in Example 1 of Japanese Patent Laid-Open No. 8-299796 (methanol synthesis catalyst B), a catalyst prepared by a method described in Example 3 of International Publication No. WO 2011/048976 (methanol synthesis catalyst C), or a catalyst prepared by a method described in Comparative Example 4 of Japanese Patent Laid-Open No. 8-299796 (methanol synthesis catalyst D) was used. The amount of the catalyst used in each of the examples and the comparative examples below was set to the same amount.

### [Example 1]

In Example 1, a production apparatus shown in FIG. 1 was used. Each condition was as shown in Table 1. That is, 81.2 kmol/h of carbon dioxide 1 and hydrogen 3 were mixed together so that the ratio (hydrogen/carbon dioxide) of the molar flow rate of the hydrogen to the molar flow rate of the carbon dioxide reached 3.05 to obtain a synthesis gas 4, and methanol was then synthesized using the synthesis gas 4. The methanol synthesis catalyst C was used as a catalyst in a methanol synthesis reactor in a methanol synthesis unit 300. A multi-tube heat exchange-type reactor was used as the methanol synthesis reactor. Regarding set conditions, the pressure of a fluid that was to come into contact with the catalyst in the reactor was set to 10.0 MPaG, the shell pressure was set to 4.0 MPaG, the circulation ratio was set to 4.0, and the temperature was 200°C to 234°C. Distillation was performed under conditions of a methanol distillation efficiency of 99% and a tower top composition of ethanol of 5 ppm. The results of the amount of methanol produced (ton/D) by Example 1 and the like are shown in Table 1.

In Example 2, an apparatus for producing methanol was configured by adding an electrolysis unit 100 such that treatment water 10 that was obtained from an organic matter decomposition treatment unit 600 and distillation wastewater 7 that was obtained from a distillation unit 400 were supplied to the electrolysis unit 100 as shown in FIG. 2. Electrolyzed hydrogen 14 obtained in the electrolysis unit 100 joined hydrogen 3 and was supplied to a synthesis gas preparation unit 200, and electrolyzed oxygen 15 was supplied to a combustion/heat recovery unit 800. The same operation as in FIG. 1 was performed except what has been described above. The results of the amount of methanol produced (ton/D) by Example 2 and the like are shown in Table 1.

In Comparative Example 1, the same operation as in Example 1 was performed except that the apparatus for producing methanol was configured without the organic matter decomposition treatment unit 600, the combustion/heat recovery unit 800, and the electrolysis unit 100 as shown in FIG. 3. The results of the amount of methanol produced (ton/D) by Comparative Example 1 and the like are shown in Table 1.

**[Table 1]**

| | Unit | Comparative Example 1 | Example 1 | Example 2 |
|---|---|---|---|---|
| Amount of methanol produced | [kg/D] | 60000 | 60290 | 60290 |
| Amount of carbon dioxide discharged by combustion of distillation waste liquid | [kg/h] | 27 | 0 | 0 |
| Amount of carbon dioxide discharged from purged gas | [kg/h] | 52.8 | 57.2 | 57.2 |
| Amount of carbon dioxide discharged to the atmosphere ¹ | [kg/h] | 79.8 | 57.2 | 57.2 |
| Total amount of unrecycled distillation waste liquid and distillation wastewater ² | [kg/h] | 1434.4 | 1538.9 | 0 |
| Amount of electrolyzed hydrogen supplied | [kmol/h] | 0 | 1.3 | 85.4 |
| Amount of hydrogen supplied other than electrolyzed hydrogen | [kmol/h] | 247.7 | 247.7 | 163.6 |
| Total amount of hydrogen supplied | [kmol/h] | 247.7 | 249 | 249 |

Regarding (*1) in Table, the amount of carbon dioxide discharged to the atmosphere means an amount changed based on an ordinary modification of the methanol synthesis process and does not refer to an amount of carbon dioxide discharged to the atmosphere from the entire process.

Regarding (*2) in Table, the total amount of unrecycled distillation waste liquid and distillation wastewater means an amount changed based on an ordinary modification of the methanol synthesis process and does not refer to an amount discharged from the entire process.

In Examples 1 and 2, the carbon collection rates from the distillation waste liquid by the anaerobic treatment were 70%.

What has been described above showed that, in Examples 1 and 2, the recycling efficiencies of the distillation waste liquid were excellent, the amounts of carbon dioxide discharged to the atmosphere were small, and the environmental loads were small compared with those in Comparative Example 1.

### Industrial Applicability

The present invention is industrially applicable in methods and apparatus for producing methanol.

The present application claims priority based on Japanese Patent Application (Japanese Patent Application No. 2023-218416), filed on December 25, 2023, the content of which is incorporated thereinto by reference.

### Reference Signs List

1 ··· Carbon dioxide-containing gas, 3 ··· hydrogen, 4 ··· synthesis gas, 5 ··· crude methanol, 6 ··· distillation waste liquid, 7 ··· distillation wastewater, 9 ··· decomposed gas, 10 ··· treatment water, 12 ··· purged gas, 13 ··· methanol, 14 ··· electrolyzed hydrogen, 15 ··· electrolyzed oxygen, 100 ··· electrolysis unit, 200 ··· synthesis gas preparation unit, 300 ··· methanol synthesis unit, 400 ··· distillation unit, 600 ··· organic matter decomposition treatment unit, 800 ··· combustion/heat recovery unit, 601 ··· microbe, 602 ··· sludge, 603 ··· returned sludge, 604 sludge dehydration unit, 605 dehydrated sludge, 610 methane generation reaction tank, 611 ··· precipitation tank, 612 ··· sludge concentration tank, 700 ··· neutralization unit, A1 to A8 ··· gasification preparation step, A201 ··· gasification unit, A202 ··· gas cleaning step, A203 ··· reforming step, A204 ··· carbon dioxide separation step, A205 ··· hydrogenation step, A206 ··· shift reaction step, A207 ··· reverse shift reaction step

## Claims

1. A method for producing methanol, comprising:
a step (A) of obtaining a synthesis gas containing at least carbon dioxide and hydrogen;
a step (B) of reacting the synthesis gas in the presence of a catalyst to obtain a methanol mixture;
a step (C) of distilling the methanol mixture to separate methanol, distillation waste liquid, and distillation wastewater, respectively; and
a step (D) of performing an organic matter decomposition treatment on the distillation waste liquid and/or the distillation wastewater to obtain decomposed gas and treatment water.

2. The method for producing methanol according to Claim 1, further comprising:
a step (E) of electrolyzing at least one of the distillation wastewater and the treatment water to obtain electrolyzed hydrogen and electrolyzed oxygen.

3. The method for producing methanol according to Claim 2, wherein the electrolyzed hydrogen is used in the step (A).

4. The method for producing methanol according to any one of Claims 1 to 3, further comprising:
a step (F) of using the decomposed gas in the step (A).

5. The method for producing methanol according to Claim 4, wherein the organic matter decomposition treatment is an anaerobic treatment.

6. The method for producing methanol according to Claim 4 or 5, further comprising:
a step (G) of combusting the decomposed gas to recover heat.

7. The method for producing methanol according to Claim 6, wherein, in the step (G), the oxygen obtained in the step (E) is used for combustion.

8. The method for producing methanol according to Claim 1, wherein the organic matter decomposition treatment is an aerobic treatment.

9. The method for producing methanol according to Claim 1, further comprising:
a step (H) of collecting carbon dioxide from the decomposed gas.

10. An apparatus for producing methanol, comprising:
a synthesis gas preparation unit;
a methanol synthesis unit;
a distillation unit; and
an organic matter decomposition treatment unit.

11. The apparatus for producing methanol according to Claim 10, further comprising:
an electrolysis unit,
wherein electrolyzed hydrogen that is obtained in the electrolysis unit is used in the synthesis gas preparation unit.
